# EUROPEAN PATENT APPLICATION

(11) **EP 1 900 321 A1**
(43) Date of publication of application: **19.03.2008**
(21) Application number: 06767435.8
(22) Date of filing: 27.06.2006
(51) Int. Cl.: A61B 5/00

(54) **NEEDLE INTEGRATING BIOSENSOR**

(30) Priority: 27.06.2005 JP 2005185988; 11.07.2005 JP 2005201072; 28.07.2005 JP 2005218685
(71) Applicant: National Institute of Advanced Industrial Science and Technology, Tokyo 100-8921 (JP)
(72) Inventor: NAKAMURA, Hideaki, NAT. INST. OF ADV. INDUSTRIAL, Tsukuba-shi, Ibaraki 305-8562 (JP); GOTOH, Masao, NAT. INST. OF ADV. INDUSTRIAL, Tsukuba-shi, Ibaraki 305-8562 (JP); KARUBE, Isao, NAT. INST. OF ADV. INDUSTRIAL, Tsukuba-shi, Ibaraki 305-8562 (JP)
(74) Representative: Cross, Rupert Edward Blount
(86) International application number: PCT/JP2006/312818
(87) International publication number: WO 2007/001001

(57) **Abstract**

There is a needle integrated biosensor, including: a biosensor including at least two electrodes, and a puncture needle to collect a body fluid by piercing a skin of a person to be tested, the biosensor and the puncture needle being integrated, wherein the puncture needle is able to be kept hygienic without the need of a protective cover or the like for the puncture needle.

There is a needle integrated biosensor, including: a biosensor including at least two electrodes, and a puncture needle to collect a body fluid by piercing a skin of a person to be tested, the biosensor and the puncture needle being integrated, wherein a soft material is attached to the front end of the puncture needle so that the front end of the puncture needle is protected.

## Description

### Technical Field

The present invention relates to a needle integrated biosensor. More particularly, it relates to a needle integrated biosensor having such a constitution that a puncture needle for collection of blood by piercing the skin and a biosensor for collection and analysis of body fluids which are taken out to the outside the skin surface are integrated.

### Background Art

Up to now, there have been cases where a patient himself/herself suffering from diabetes mellitus collects his/her blood and measures the blood sugar level which is a glucose content in blood. In that case, the patient uses a blood collecting instrument called a lancet where a blood collecting needle is attached and detached to collect the blood by piercing his/her finger or arm and transfers the collected blood to a blood sugar level analyzer to measure the blood sugar level. According to such a measuring system, the patient must carry a set of measuring instrument comprising several items such as blood sugar level analyzer, lancet, blood collecting needle and analyzing element and conduct the measurement by assembling them upon necessity. Thus, a long period of training is needed for its operation and considerable time is required until a reliable measurement is able to be conducted by the patient himself/herself. In fact, measurement at the site other than fingers and forearms (such as abdominal wall and earlobe) is difficult for even skilful persons. In recent years, due to the needs for providing a minimally invasive sample having less pain, a biosensor by which measurement is possible where the amount of the sample is 1 µl or less has been developed and, in the case of such a very small amount, a work for a correct supply of a sample to a biosensor is very difficult. As a result, a error in the measurement is resulted and there are inconveniences that the patient who is a person to be measured must be punctured once again and that a biosensor must be also exchanged to make a fresh start for the measurement.
Patent Document 1: Japanese Patent Laid-Open No.Hei09-266898
Patent Document 2: Examined Japanese Patent Application Publication No.Hei08-02.0412

Under such circumstances, various needle integrated biosensors have been devised. Firstly, in a needle integrated biosensor mentioned in the Patent Document 3, each of a puncture needle and a biosensor is set at different positions in an inner area of a measuring apparatus of a pen type (like a two-colored ball point pen) equipped with a driving part for a puncture needle and, after the front end of the pen-shaped measuring apparatus is applied to the skin of a person to be tested and pierced, a biosensor is exposed at the front end to collect the blood whereupon measurement of blood sugar level is conducted. In this method however, troublesomeness that each of the needle and the biosensor must be set onto a measuring apparatus has not been solved.
Patent Document 3: Japanese Patent Laid-Open No. 2000-217804

In another needle integrated biosensor as mentioned in the Patent Document 4, a puncture needle is subjected to driving from outside and there is adopted an integrated structure where a puncture needle moves parallel along a longitudinal direction of a slender biosensor in a small piece. However, in such a type, since a puncture needle is exposed from a biosensor, a cover which protects the front end of the puncture needle is necessary and, further since the puncture needle moves in a path for collection and conveyance of the blood and in a reagent layer, there is a risk that the surface of the puncture needle is contaminated by a reagent before the puncture needle pierces the skin of the person to be tested. Moreover, in such a type, since the puncture needle is exposed from the biosensor, a cover for protecting the front end of the puncture needle is necessary and, further, there is no other way than to utilize a capillary phenomenon for sending the collected blood after piercing to the biosensor.
Patent Document 4: Japanese Patent Re-Laid-Open No. 2002-056769

Still further, in the conventional needle integrated biosensor, the structure is complicated, amount of the collecting blood necessary as a sample liquid is too much and there is an affection by intake into unnecessary spaces.

### Disclosure of the Invention

### Problems to be solved by the Invention

An object of the present invention is to provide a needle integrated biosensor where, in a biosensor in which a biosensor equipped with at least two electrodes and a puncture needle for collection of body fluids by piercing the skin of a person to be tested are integrated, the puncture needle is able to be kept hygienic without a protective cover or the like for the puncture needle.

Another object of the present invention is to provide a needle integrated biosensor by which an efficient sending of the collected blood after piercing is able to be made possible.

### Means for solving the Problems

The object of the present invention as such is able to be achieved by a needle integrated biosensor where, in a biosensor in which a biosensor equipped with at least two electrodes and a puncture needle for collection of body fluids by piercing the skin of a person to be tested are integrated, a soft material is attached to the front end of the puncture needle whereby the front end of the needle is protected.

Another object of the present invention is able to be achieved by a needle integrated biosensor where, in a biosensor having such a constitution that a biosensor equipped with electrode and spacer and a puncture needle for collection of body fluids by piercing the skin of a person to be tested aligned in the biosensor are integrated by means of a puncture needle support in a space between two electrically insulating substrates, a piercing and blood-collecting opening at the front end of the puncture needle is tightly closed by a puncturable soft material while, in the rear end of the puncture needle, space between two substrates and the puncture needle support is tightly closed by a shrinkable material and the inner area of the biosensor is kept in a negative pressure whereby blood is collected by suction and the components to be detected are measured when the puncture needle breaks through both of the soft material and the skin by being driven from outside.

Still another object of the present invention is able to be achieved by a needle integrated biosensor where, in a biosensor having such a constitution that a biosensor equipped with electrode and spacer and a puncture needle for collection of body fluids by piercing the skin of a person to be tested aligned in the biosensor are integrated by means of a puncture needle support in a space between two electrically insulating substrates, each of the above substrates is equipped with a cutting part by which separation of substrate part where no electrode is formed, a piercing and blood-collecting opening at the front end of the puncture needle is tightly closed by a puncturable soft material, cutting parts of the two substrates are covered by a shrinkable material equipped at the rear end of the puncture needle and tightly close the space between the two substrates and the puncture needle so that the inner area of the biosensor is tightly closed and, after the puncture needle breaks through both of the soft material and the skin at the same time by being driven from outside, a part of the substrates is separated at the above cutting part and the volume of the inner area of the biosensor is increased to make the inner area negative pressure whereby blood is collected by suction and the components to be detected are measured.

### Effects of the Invention

In a needle integrated biosensor in accordance with the present invention, a soft material is attached to the front end of the puncture needle so that the front end of the needle is protected whereby there is achieved an excellent effect that the puncture needle is able to be kept hygienic until actual use. Moreover, in the needle integrated biosensor having such a constitution, no material such as a protective cover for the puncture needle is necessary and, therefore, its operation is excellent and reduction of the manufacturing cost is also able to be achieved.

Further, in a needle integrated biosensor in accordance with the present invention, a suction means is used together with a capillary phenomenon in sending the collected blood into a biosensor and, therefore, the collected blood after piercing is able to be efficiently sent into the biosensor. Still further, since the puncture needle is included in the biosensor, no cover which protects its front end is necessary and that is appreciated in terms of safety as well. Furthermore, when the puncture needle is aligned in parallel to the long-axial direction of the electrode, contact of the puncture needle to reagent layer, etc. formed on the electrode is able to be prevented whereby it is now possible to avoid the inconvenience such as pollution of the puncture needle with the reagent, etc.

In addition, since a suction means in addition to the capillary phenomenon is used in sending the collected blood into the biosensor in accordance with the needle integrated biosensor of the present invention, the collected blood after piercing is able to be efficiently sent into the biosensor. Moreover, since the puncture needle is included in the biosensor, no cover for protecting its front end is necessary and that is appreciated in terms of safely as well.

Further, when the puncture needle is aligned in such a mode of being crossed at right angles to the long-axial direction of the electrode, there is achieved an excellent effect that an efficient measurement is possible without collection of blood which is in more than the necessary amount after piercing. Furthermore, in the case of a shape where are bilaterally asymmetric to the puncture needle which is the central line, a wrong insertion into the measuring apparatus in use is able to be prevented.

### Brief Description of the Drawings

Fig. 1 is a drawing which shows an example of assembling of a needle integrated biosensor according to the present invention.
Fig. 2 is a drawing which shows another example of assembling of a needle integrated biosensor according to the present invention.
Fig. 3 is a drawing which shows an example of constitution of a needle integrated biosensor according to the present invention.
Fig. 4 is a drawing which shows an example of use of a needle integrated biosensor according to the present invention.
Fig. 5 is a drawing which shows an example of assembling of a needle integrated biosensor according to the present invention.
Fig. 6 is a drawing which shows an example of constitution of a needle integrated biosensor according to the present invention.
Fig. 7 is a drawing which shows an example of use of a needle integrated biosensor according to the present invention.
Fig. 8 is a drawing which shows an example of constitution of a measuring apparatus equipped with a piercing drive for a needle integrated biosensor according to the present invention.
Fig. 9 is a drawing which shows an example of assembling of a needle integrated biosensor according to the present invention.
Fig. 10 is a drawing which shows another example of assembling of a needle integrated biosensor according to the present invention.
Fig. 11 is a drawing which shows an example of constitution of a needle integrated biosensor according to the present invention.
Fig. 12 is a drawing which shows an example of use of a needle integrated biosensor according to the present invention.
Fig. 13 is a drawing which shows still another example of assembling of a needle integrated biosensor according to the present invention.

### Explanation of referential numerals

- 1A: substrate
- 2A: spacer
- 3A: needle integrated biosensor
- 4A: penetration hole
- 5A: adhesive layer
- 6A: resist layer
- 7A: electrically conductive material
- 8A: sample-conveying/puncture-needle path
- 10A: electrode
- 11A: terminal
- 12A: opening for piercing and collecting blood
- 13A: electrode reactive part
- 14A: puncture needle part
- 15A: soft material
- 17A: connecting part to outer drive
- 19A: support for puncture needle
- 20A: puncture needle
- 24A: collection of blood
- 1B: lower substrate
- 2B: spacer
- 3B: needle integrated biosensor
- 4B: penetration hole
- 5B: adhesive layer
- 6B: resist layer
- 7B: electrically conductive material
- 8B: sample-conveying/puncture-needle path
- 9B: upper substrate
- 10B: electrode
- 11B: terminal
- 12B: opening for piercing and collecting blood
- 13B: electrode reaction part (reagent layer)
- 14B: puncture needle part
- 15B: soft material
- 16B: shrinkable material
- 17B: connecting part to outer drive
- 19B: support for a puncture needle
- 20B: puncture needle
- 23B: adhesive part
- 24B: collection of blood
- 25B: pressing device
- 26B: measuring apparatus where a piercing drive is installed
- 27B: introducing part for a needle integrated biosensor
- 28B: trigger part
- 29B: operation panel
- 30B: indication part
- 31B: operation button
- 33B: piercing initiation button
- 34B: anti-sliding device
- 35B: hook
- 1C: substrate
- 2C: spacer
- 3C: needle integrated biosensor
- 4C: penetration hole
- 5C: adhesive layer
- 6C: resist layer
- 7C: electrically conductive material
- 8C: sample-conveying/puncture needle path
- 10C: electrode
- 11C: terminal
- 12C: opening for piercing and collecting blood
- 13C: electrode reaction part (reagent layer)
- 14C: puncture needle part
- 15C: soft material
- 16C: shrinkable material
- 17C: connecting part to outer drive
- 18C: folding molded material
- 19C: support for a puncture needle
- 20C: puncture needle
- 21C: connecting part
- 22C: hook
- 23C: adhesive part
- 24C: collection of blood
- 25C: pushing device
- 26C: cutting part

### Best Mode for Carrying Out the Invention

As to a substrate, an electrically insulating one is sufficient and, for example, plastic, biodegradable material, paper, etc. is used therefor and, preferably, polyethylene terephthalate is used therefor. In the substrate, a cutting part is formed at the area after a puncture needle from the electrode forming site. The cutting part is formed as an easily cuttable binding part such as at least one linear binding part between the substrates or, preferably, a linear binding part where the central area is constricted or is constituted as a groove in a depth which does not penetrate the substrate at least on one side of the substrate or, preferably, as a V-shaped groove. As to the linear binding part, that having a width of 0.2 to 2 mm or, preferably, 0.5 to 1 mm and a length of 0.2 to 2 mm or, preferably, 0.5 to 1 mm is used specifically. Such cutting parts are formed on the same position when two substrates are aligned oppositely.

The electrode is formed on a substrate by a screen printing method, a vapor deposition method, a sputtering method, a foil adhesion method, a galvanizing method, etc. and, as to a material therefor, carbon, silver, silver/silver chloride, platinum, gold, nickel, copper, palladium, titanium, iridium, lead, tin oxide and platinum black may be exemplified. As to the carbon hereinabove, carbon nanotube, carbon microcoil, carbon nanohone, fullerene, dendrimer or a derivative thereof may be used.

The electrode may be a two-electrode method formed by working electrode and counter electrode, a three-electrode method formed by working electrode, counter electrode and reference electrode or an electrode method formed by more electrode numbers. When a three-electrode method is adopted, a transfer speed of collected blood introduced into a conveying path is able to be measured in addition to an electrochemical measurement of the substance to be measured whereby a hematocrit value is able to be measured. It is also possible to constitute from two or more sets of electrode system. Those electrodes are formed together on a sheet of substrate or separately on two sheets of substrate.

Although those electrodes are formed together on a sheet of substrate or separately on two sheets of substrate, a facing structure where electrode is oppositely aligned on two substrates or, to be more specific, a facing structure where electrodes formed on the surfaces of two substrates sandwich a spacer comprising resist layer or adhesive layer is preferred from the viewpoint of making the sample volume small. As a result thereof, electrochemical reaction proceeds efficiently and volume of reaction layer can be effectively made small by shortening of distance between electrodes and of electrode area whereby it is possible to make the amount of the sample small.

A reagent layer (electrode reaction part) is able to be formed on a substrate where electrode is formed. The reagent layer is formed by a screen printing method or a dispenser method and fixation of the reagent layer onto the electrode surface or the substrate surface is able to be conducted by a covalent bond method or an adsorbing method accompanied by drying. With regard to a reagent aligned to the electrode reaction part of the biosensor, that which contains glucose oxidase which is an oxidizing enzyme and potassium ferricyanide as a mediator may be exemplified when constitution is done for the measurement of blood sugar level. When the reagent is dissolved in blood, an enzymatic reaction starts and, as a result, potassium ferricyanide coexisting in the reaction layer is reduced and potassium ferrocyanide which is an electron transmitter of a reduced type is accumulated. Its amount is proportional to the substrate concentration or, in other words, the concentration of glucose in blood. The electron transmitter of a reduced type which is accumulated for a predetermined period is oxidized by electrochemical reaction. An electron circuit in the main body of the measuring apparatus which will be mentioned later calculates and determines the glucose concentration (blood sugar level) from the positive electrode current measured at that time and displays it onto the display aligned on the surface of the main body.

Surfactant and lipid may also be applied around the opening for collection of blood and on the surface of electrode or reagent layer (electrode reaction part). As a result of application of the surfactant and the lipid, it is now possible to make the transfer of the sample smooth.

At that time, as a result of application of reagent layer, surfactant or lipid into the conveying path of the sample, there is a possibility that a puncture needle contained therein is contaminated. In order to prevent the pollution as such, it is preferred that such a reagent is not applied around the front end of a puncture needle.

In the biosensor where a reagent layer is equipped on the electrode where the above blood collection is satisfied, the collected blood reacts with the reagent when the collected blood sent from the opening for blood collection contacts to the reagent layer on the electrode. This reaction is monitored as electric changes in the electrode.

It is also possible that the electrode of the biosensor is provided by a resist layer and the resist layer is also able to be easily formed by a screen printing or the like. Like an adhesive, there is no particular limitation for the resist in that case provided that it does not react with or is not dissolved in the substrate and its examples are an ultraviolet-hardening vinyl-acrylic resin, urethane-acrylic resin and polyester-acrylic resin. Main object for the use of a resist is to make the electrode pattern clear and to clarify the stipulation of the above electrode area and other object is to insulate the sample-conveying path where no reagent layer is present. For that purpose, the resist layer may or may not form the same pattern as the adhesive layer. In the latter case, it is preferred that the resist layer is formed on an electrode substrate for the sake of insulation. Further, when the resist layer is formed thicker than the electrode in a sample-conveying path where the puncture needle of the needle integrated biosensor according to the present invention is received, contact of the puncture needle to the electrode is able to be suppressed. Such a resist layer is also able to be formed by a screen printing method and, for example, a resist layer formed by any of the above materials in the thickness of about 50 to 500 µm or, preferably, about 10 to 100 µm also acts as a spacer.

Two substrates are adhered by an adhesive such as that of an acrylic resin type to constitute a biosensor. Such an adhesive layer is also able to be formed by a screen printing method, is formed in a thickness of about 5 to 500 µm or, preferably, about 10 to 100 µm and, like a resist layer and acts as a spacer as well. It is also possible that the above reagent is contained in the adhesive layer. The adhesive layer may be in a pattern which is same as or is different from the resist layer.

When the substrate having the above constitution in which a puncture needle is installed is folded along a connecting part, a biosensor as a folded molding product is able to be manufactured. As to the connecting part, that having a length of not shorter than the thickness of a spacer or, 0.5 to 4 mm and, preferably, 1.0 to 3. 0 mm is placed preferably between the two substrates in at least two places. The connecting part as such in a length of about 0.5 to 0.9 mm is able to be formed as a perforation in a broken line on an insulating substrate using, for example, a cogwheel-shaped thin disk where its convex part is a blade while, in the case of a connecting part in a length of about 1 to 4 mm, the insulating substrate is punched with a mold to subject to a hinge molding. When the connecting part is in a length of about 1 to 4 mm, it is not necessary that the folded area is subj ected to a thermal adhesion with pressure or fixed using a fixing device so as to prevent a backward bending. In the case of a biosensor which is a folded molding product as such, it is possible to manufacture a lot of biosensors at a time by such a means that a connecting part as a folding line is formed in a horizontal way to a direction of long axis of a long substrate, then electrode, etc. are formed, folding along the connecting part is conducted and punching into a shape of a sensor is done. In a needle integrated biosensor which is manufactured by such a method, reproducibility is also very good and it has advantages which have not been achieved in the conventional layered method.

In a sample-conveying path of a biosensor, there is installed a puncture needle which is, for example, in parallel to the direction of long axis of electrode and pierces the skin of a person to be tested so as to collect the body fluid.

With regard to a puncture needle for collecting the body fluid from the skin of a person to be tested, since it is necessary to pass through the soft material and further pierce the body to be tested, it is desired to have strength durable the above and to be sharp and, furthermore, it is to be a fine puncture needle for suppressing the pain upon piercing. To be more specific, that made by Terumo Corporation and being 21 to 33 gauges is used. The puncture needle may be either hollow or rod-shaped so far as it is able to break through the skin of the person to be tested. Since the puncture needle is to be hygienically received in a biosensor until its actual use, a photocatalytic function effective for antibiotic and antiviral purpose may be applied to the surface of the front end of the needle. In that case, a membrane of titanium oxide or titanium dioxide is preferred.

In a biosensor, a puncture needle for piercing the skin of the person to be tested and to collect the body fluid is installed. Although the puncture needle may be in any alignment such as in parallel or at right angles to the electrode, it is placed in a state of crossing the electrode at the angles of 30 to 90° or, preferably, at right angles. When a puncture needle is placed in a state of being right angles to the electrode, amount of the collecting blood is able to be suppressed as compared with the case where the puncture needle is placed in parallel to the long axial direction of the electrode and, moreover, when the terminal for measurement is placed at the position which is apart from the orbit of the puncture needle, the shape of the needle integrated biosensor is bilaterally asymmetric along the puncture needle as a central line whereby a user is able to adopt it as a mark so that an error for right and left in inserting into a measuring apparatus is able to be avoided and the measuring apparatus is also able to be equipped with a mechanism for specifying the position of the measuring terminal of the needle integrated biosensor according to the present invention.

A soft material is attached to the front end or the puncture needle or, to be more specific, at least to the area contacting to the body to be tested. As a result, the front end of the needle is protected. Until a step where a puncture needle is subjected to a piercing drive and to breaking through the skin of the person to be tested, it is necessary that the soft material does not attach to the surface of the puncture needle and that pieces thereof, etc. are not mixed with the collected blood and, therefore, it is fixed on at least a part of a biosensor such as a substrate using an adhesive of an acrylate type.

Examples of such a soft material are gel-like material, elastic material and foamed material and, in the case of a foamed material, an example thereof is an artificial skin which is very similar to the skin to be pierced and, preferably, is able to be sterilized. As to a sterilizing method, there may be used sterilization with ethanol, sterilization by dry heating, autoclave, ultraviolet ray, gamma ray, etc. and, for example, agar which is one of the gel-like materials is sterilized by an autoclave, cooled and applied to the front end of a needle before gelling.

It is also possible to give an antibiotic action to the soft material. As to a material having an antibiotic action, metal such as copper and silver has been known and a soft material having an antibiotic action is prepared when small amount of such a metal is contained in a soft material.

During the manufacture of a biosensor of the present invention, it is constituted in such a manner that, under a condition of negative pressure than the outer air or, preferably, under a vacuum condition, a space between a support for a puncture needle and two substrates constituting the biosensor at the side of a piercing drive is constituted so as to give a tightly closed state by a shrinkable material such as natural rubber whereby the inner area of the sensor is tightly closed in a negative pressure state and, in the transfer of the collected blood to a sample-conveying path after piercing, a suction means is able to be used in addition to a capillary phenomenon. At that time, when a puncture needle is further pulled in the direction opposite to the piercing direction immediately after the piercing, the shrinkable material is extended and the collected blood is able to be sucked under the state where the inner negative pressure is further enhanced. As a result of adoption of the constitution as such, it is now possible to smoothly conduct the collection of the blood.

Further, an opening for collection of blood by piercing in the biosensor of the present invention is covered by a soft material such as silicone rubber, soft polyurethane, polyvinyl chloride and foamed styrene and, in the piercing driving side, its constitution is in such a manner that the space between s support for a puncture needle and two substrates constituting the biosensor is made tightly closed by a shrinkable material (shrinkale agent) such as natural rubber. During the manufacture of the biosensor as such, the inner area of the sensor is tightly closed under the condition of negative pressure than the outer air or, preferably, under a vacuum condition whereby the inner area of the sample-conveying path of the biosensor is made in negative pressure than the outer air pressure. As such, in the needle integrated biosensor according to the present invention, a suction means is used in addition to a capillary phenomenon for transfer of the collected blood to a sample-conveying path so that the collection of blood after piercing is conducted smoothly. At that time, when a puncture needle is further pulled in the direction opposite to the piercing direction immediately after the piercing, the shrinkable material is extended and the collected blood is able to be sucked under the state where the inner negative pressure is further enhanced.

Further, an opening for collection of blood by piercing in the biosensor of the present invention is covered by a soft material such as silicone rubber, soft polyurethane, polyvinyl chloride and foamed styrene and, in the piercing driving side, its constitution is in such a manner that the space between s support for a puncture needle and two substrates constituting the biosensor including a cutting part is made tightly closed by a shrinkable material such as natural rubber. As a result of the above constitution, inner area of the sensor is tightly closed and, with regard to transfer of the collected blood after piercing to a sample-conveying path, a suction means is able to be used in addition to a capillary phenomenon whereby it is now possible to collect the blood smoothly. Here, in the needle integrated biosensor according to the present invention, the terminal is able to be made outside of the orbit of the puncture needle as mentioned above and, therefore, a structure for keeping the air tightness of the inner area of the sample-conveying path including the puncture needle is able to be easily available. The soft material covering the opening for piercing and collecting the blood keeps the tightly closed state and also has an effect of improving the close adhesion of the skin of the person to be tested and the opening for piercing and collecting the blood.

It is desirable that the needle integrated biosensor according to the present invention is able to conduct a series of operations of piercing, blood collection and measurement by means of a measuring apparatus equipped with a piercing drive. In that case, with regard to a piercing drive for example, it is preferred to be equipped with a mechanism where the needle pierces the soft material of the biosensor and breaks through the skin of the person to be tested and a mechanism where it quickly returns to the original position immediately after piercing.

Further, when the needle integrated biosensor according to the present invention is equipped with a sucking mechanism, the piercing drive system in the measuring apparatus may be still improved so as to enhance the sucking force upon collection of the blood. Thus, the following constitution may be adopted that, when the puncture needle is further pulled in the direction which is opposite to the piercing direction using a mechanism in which the puncture needle is returned to the original position immediately after piercing, a shrinkable material is extended and the negative pressure in the inner area is made stronger.

In the needle integrated biosensor according to the present invention, it is desirable that the biosensor is set in a measuring apparatus equipped with a piercing drive whereby a series of operations of piercing, blood collection and measurement is carried out. In that case, it is desirable that the piercing drive, for example, is equipped with a mechanism where a needle pierces the soft material of the biosensor and breaks through the skin of the person to be tested and a mechanism where it quickly returns to the original position immediately after the piercing.

It is also possible that a piercing drive system in the measuring apparatus is further improved so as to enhance the sucking force by a needle integrated biosensor upon collection of the blood. Thus, it is also possible that, when the puncture needle is further pulled to the direction which is opposite to the piercing direction using a mechanism for returning the arrangement of puncture needle to the original position immediately after piercing, the shrinkable material is extended as mentioned above so that the negative pressure in the inner area is made stronger.

It is desirable that, in the needle integrated biosensor according to the present invention, a series of operations of piercing, blood collection and measurement is able to be conducted by a measuring apparatus equipped with a piercing drive. In that case, it is desirable that the piercing drive, for example, is equipped with a mechanism where a needle passes through a soft material of a biosensor to break through the skin of a person to be tested and a mechanism where it quickly returns to the original position immediately after the piercing.

Further, in order to enhance the sucking force upon collection of the blood, the piercing drive system in the measuring apparatus is equipped with a mechanism where a cutting part is separated by further pulling of the puncture needle in a direction which is opposite to the piercing direction using a mechanism of returning the arrangement of the puncture needle to the original position immediately after the piercing whereby the shrinkable material covering the cutting part is extended and the inner area of the biosensor becomes negative pressure.

With regard to a measuring apparatus for a needle integrated biosensor, there is used an apparatus in which operability and durability for surely and repeatedly conducting the measurement using the needle integrated biosensor are ensured and which is able to be easily carried and there is also used a measuring apparatus having such a function that a state where the measurement is possible is resulted when a needle integrated biosensor is inserted into an introducing part on the lower area whereby the support for a puncture needle turns upside and the terminal of the biosensor is connected to a connector of the measuring apparatus, then preparation of the measurement is completed when a trigger is pulled so as to give a piercing drive to the inner part of the needle integrated biosensor, then piercing, blood collection and measurement are automatically done in this order by pushing the switch of the piercing initiation button and the measuring result is induced finally.

An example of the characteristics of the measuring apparatus will be mentioned in more detail. In this measuring apparatus, a driving part for a puncture needle and a measuring apparatus part are integrated and the driving part for a puncture needle is constituted from a trigger part, a piercing initiation button part and a driving part made of elastic substances such as spring. On the other hand, in the measuring apparatus part, its fundamental constitution comprises a sensor introducing part, a connector, a circuit for electrochemical measurement, a memory part, an operation panel, a measuring part which measures electric value of the biosensor at the electrode and a display part which displays the measured value at the measuring part and is further able to be installed with electric wave as a wireless means such as Blue Tooth (registered trade mark). Due to such a slide structure, a piercing drive is applied keeping the state where the needle integrated biosensor is surely held whereby the strength of the measuring apparatus as a whole is able to be enhanced. The measuring apparatus is also able to be equipped with a mechanism which is able to recognize the asymmetric structure where a puncture needle of a needle integrated biosensor is a central line at the projected part of the terminal for the measurement.

Further, with regard to a measuring apparatus for a needle integrated biosensor, there is used an apparatus in which operability and durability for surely and repeatedly conducting the measurement using the needle integrated biosensor are ensured and which is able to be easily carried and there is also used a measuring apparatus having such a function that a state where the measurement is possible is resulted when a needle integrated biosensor is inserted into an introducing part on the lower area whereby the support for puncture needle turns upside and the terminal of the biosensor is connected to a connector of the measuring apparatus, then preparation of the measurement is completed when a trigger is pulled so as to give a piercing drive to the inner part of the needle integrated biosensor, then piercing, blood collection and measurement are automatically done in this order by pushing the switch of the piercing initiation button and the measuring result is induced finally.

An example of the characteristics of the measuring apparatus will be mentioned in more detail. In this measuring apparatus, a driving part for a puncture needle and a measuring apparatus part are integrated and the driving part for a puncture needle is constituted from a trigger part, a piercing initiation button part and a driving part made of elastic substances such as spring. On the other hand, in the measuring apparatus part, its fundamental constitution comprises a sensor introducing part, a connector, a circuit for electrochemical measurement, a memory part, an operation panel, a measuring part which measures electric value of the biosensor at the electrode and a display part which displays the measured value at the measuring part and is further able to be installed with electric wave as a wireless means such as Blue Tooth (registered trade mark). Due to such a slide structure, a piercing drive is applied keeping the state where the needle integrated biosensor is surely held whereby the strength of the measuring apparatus as a whole is able to be enhanced.

Further, with regard to a measuring apparatus for a needle integrated biosensor, there is used an apparatus in which operability and durability for surely and repeatedly conducting the measurement using the needle integrated biosensor are ensured and which is able to be easily carried and there is also used a measuring apparatus having such a function that a state where the measurement is possible is resulted when a needle integrated biosensor is inserted into an introducing part on the lower area whereby the support for puncture needle turns upside and the terminal of the biosensor is connected to a connector of the measuring apparatus, then preparation of the measurement is completed when a trigger is pulled so as to give a piercing drive to the inner part of the needle integrated biosensor, then piercing, blood collection and measurement are automatically done in this order by pushing the switch of the piercing initiation button and the measuring result is induced finally.

An example of the characteristics of the measuring apparatus will be mentioned in more detail. In this measuring apparatus, a driving part for a puncture needle and a measuring apparatus part are integrated and the driving part for a puncture needle is constituted from a trigger part, a piercing initiation button part and a driving part made of elastic substances such as spring. On the other hand, in the measuring apparatus part, its fundamental constitution comprises a sensor introducing part, a connector, a circuit for electrochemical measurement, a memory part, an operation panel, a measuring part which measures electric value of the biosensor at the electrode and a display part which displays the measured value at the measuring part and is further able to be installed with electric wave as a wireless means such as Blue Tooth (registered trade mark). Due to such a slide structure, a piercing drive is applied keeping the state where the needle integrated biosensor is surely held whereby the strength of the measuring apparatus as a whole is able to be enhanced. The measuring apparatus is also able to be equipped with a mechanism which is able to recognize the bilaterally asymmetric structure where a puncture needle of a needle integrated biosensor is a central line at the projected part of the terminal for the measurement.

With regard to a piercing drive of the measuring apparatus, a mechanism where, after the upper part of the needle integrated biosensor is struck in the vertical direction, it quickly returns and it is further preferred that the drive has a mechanism where depth of piercing the skin of the persons to be tested is able to be adjusted.

The measuring apparatus is also able to be equipped with a voice guidance function and a voice recognizing function corresponding to the visual impairment caused by diabetes mellitus, a control function for measured data by incorporation of a radio clock, a communication function of measured data, etc. to medical organizations, etc., a charging function and others.

Although there is no particular limitation for the measuring method at the measuring part of the measuring apparatus, there may be used potential step chronoamperometry, coulombmetry, cyclic voltammetry, etc.

In view of the above, the needle integrated biosensor in accordance with the present invention is able to cope with a universal plan where the users are not restricted.

Now each of the needle integrated biosensors in the embodiments according to the present invention will be illustrated in detail by referring to drawings and the present invention is not limited to the following examples so far as they are within a gist of the present invention.

### Examples

### Example 1

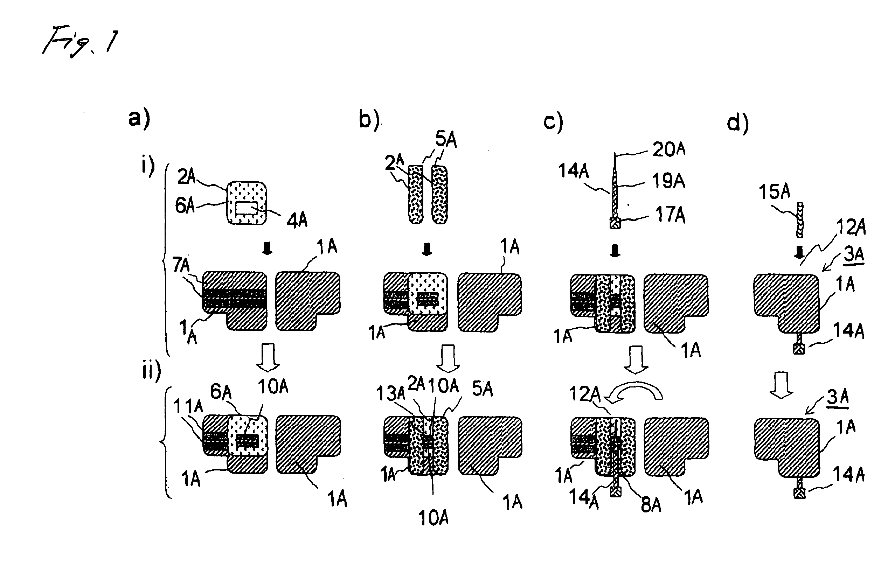

Fig. 1 is an example showing an assembling of the needle integrated biosensor according to the present invention. Figs. 1a) to d) are examples of manufacture of the needle integrated biosensor; i) shows constituting materials required for the manufacture of the needle integrated biosensor and ii) shows an examples of its molding. Fig. 1a) shows a plate material and a resist layer 6A of a substrate 1A where an electrically conductive material 7A is formed. The resist layer 6A plays a role of a spacer 2A and also regulates the electrode area and it is also installed for preventing the contact of electrode surface to puncture needle. Therefore, a penetration hole 4A is formed on the resist layer 6A. Here, two substrates 1A and 1A are made to be safely used by rounding their corners. Fig. 1b) shows the state where an adhesive layer 5A is formed on the resist layer 6A. Since the adhesive layer 5A is also formed between the two substrates 1A and 1A, it also plays a role of a spacer 2A like the resist layer 6A. Fig. 1b)ii) shows an electrode 10A where its area is regulated by the resist layer 6A and the adhesive layer 5A as well as electrode reaction part 13A thereof. A reagent layer is formed on the electrode of this electrode reaction part 13A if necessary. Fig. 1c)i) shows a constitution of a puncture needle part 14A. Thus, the puncture needle part 14A is constituted from a puncture needle 20A and a support 19A for the puncture needle as well as a connecting part 17A to an outer drive and, when the connecting part 17A to the outer drive is connected to a measuring apparatus equipped with a piercing drive, a piercing drive from the measuring apparatus is available. From Fig. 1c), there is shown a state where a puncture needle part 14A is aligned along the sample-conveying path 8A. As the drawing shows, the puncture needle part 14A has a structure where contact to the electrode surface 10A is able to be avoided by formation of the resist layer 6A. Accordingly, when a reagent layer 13A is formed on the surface of an electrode 10, contact of the reagent layer 13A to the puncture needle part 14A is able to be avoided and, as a result, pollution of the puncture needle 20A by the reagent is able to be prevented. Fig. 1d)i) shows the state where a soft material is inserted into the puncture needle part 14A of the needle integrated biosensor 3A formed as such. In that case, after gel or liquid having fluidity such as a heated agar liquid is infused into a needle integrated biosensor 3A, the soft material 15A is attached in a form of enclosing the front end of the puncture needle part 14A and is fixed in a state of protecting the front end of the needle. Thus, Fig. 1 shows the case where a soft material 15A having fluidity is introduced into a biosensor.

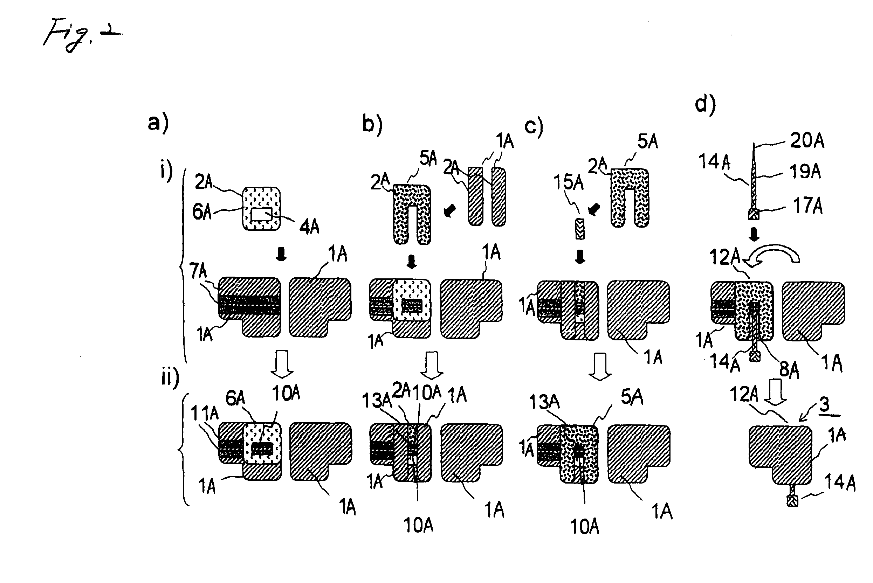

Fig. 2 shows another assembling example of a needle integrated biosensor according to the present invention. Thus, unlike Fig. 1, Fig. 2 shows the case where a soft material 15A in a solid state is placed on a substrate and then a front end of the puncture needle part 14A is pierced into the soft material 15A whereby a soft material 15A is attached to the front end of the needle so that a needle integrated biosensor 3A is manufactured. In a step of Fig. 2b), after a pattern of an adhesive layer 5A is applied so that the space between the opening 12A for piercing and collecting the blood and a penetration hole 4A of the resist layer 6A is also filled, a substrate 1A having the same pattern as the spacer 2A shown in Fig. 1b) is used as a spacer 2A and is layered on a pattern of the adhesive layer 5A. Fig. 2c) shows a state where a soft material 15A in a solid state is placed as if it fills the space between the opening 12 for piercing and collecting the blood and the penetration hole 4A of a resist layer 6A and then, for adhering the upper substrate 1A, the adhesive layer 5A having the same pattern as shown in Fig. 2b) is layered. Fig. 2d) shows the state where the front end of the puncture needle part 14A is pierced in the soft material 15A and a soft material 15A is attached to the front end of the puncture needle part 14A whereby a needle integrated biosensor 3A where the front end of a needle is protected is manufactured.

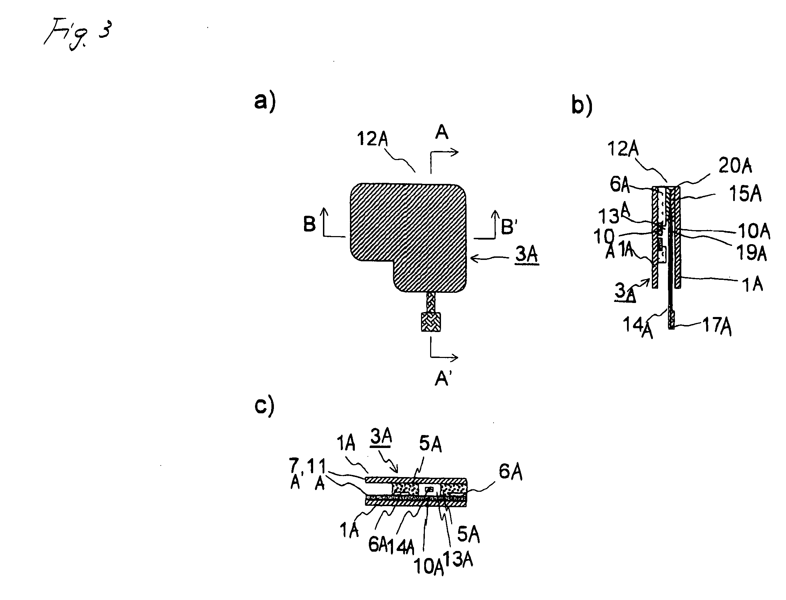

Fig. 3 shows a cross-sectional view of the constitution of the needle integrated biosensor as shown in Fig. 1. Fig. 3b) is a cross-sectional view thereof along the line A-A' of Fig. 3a). As the drawing shows, a puncture needle part 14A is placed in such a manner that a soft material 15A is attached to the front end of the puncture needle part 14A onto the pattern surface formed on the substrate 1A of the biosensor to protect the front end of the needle. Fig. 3c) shows a cross-sectional view along the line B-B' as shown in Fig. 3a). A puncture needle 14A is placed at the central part of the two substrates 1A and 1A and its front end is protected by a soft material.

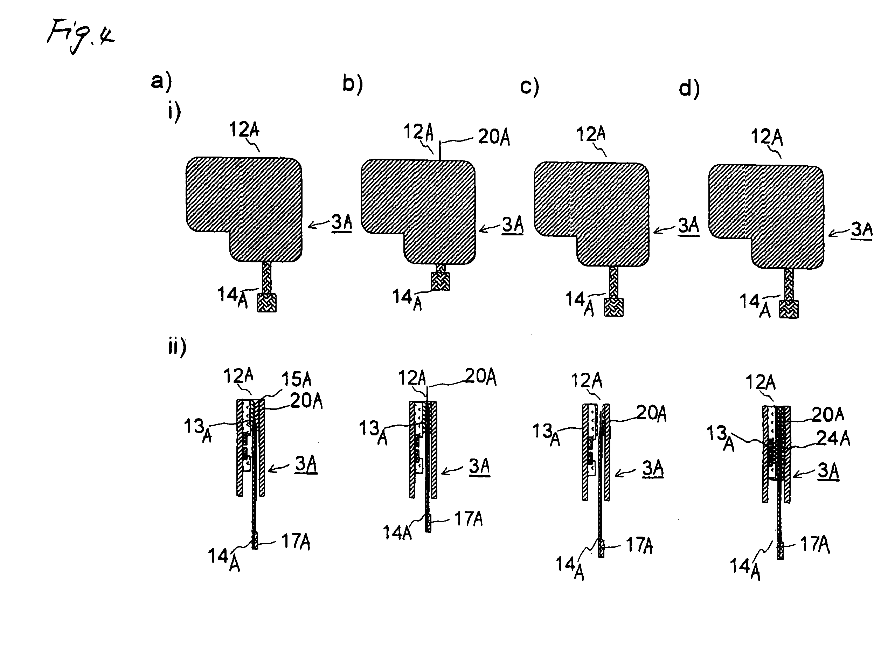

Fig. 4 shows a use example of the needle integrated biosensor 3A as shown in Fig. 1. In Fig. 4, a) to d) shows each of the steps and, with regard to i) and ii), the state of the needle integrated biosensor 3A at that time is shown in i) by means of a constitution drawing while, in ii), it is shown by a cross-sectional view along the line A-A' as shown in Fig. 3a). Fig. 4a) shows the state of the needle integrated biosensor 3A connected to a measuring apparatus with a piercing drive before actual use. At that time, the skin as a thing to be tested is closely attached to the opening 12 for collection of the blood of the needle integrated biosensor 3A. Fig. 4b) shows the state of being pierced and a state where the front end of the puncture needle 20A is projected to outside of the sensor is shown. At that time, a soft material 15A placed to the opening 12A for piercing and collecting the blood is penetrated by a puncture needle 20A and, although not shown in the drawing, the puncture needle 20A also pierces the skin. Fig. 4c) shows a state where the puncture needle part 14A returns to the original position after piercing and, in the soft material 15A, a penetration hole is opened in the piercing direction of the needle. Fig. 4d) shows the state where, after that, the collected blood 24A from the pierced skin is sent to an electrode reaction part 13A by capillary phenomenon via a penetration hole opened in the soft material 15A.

### Example 2

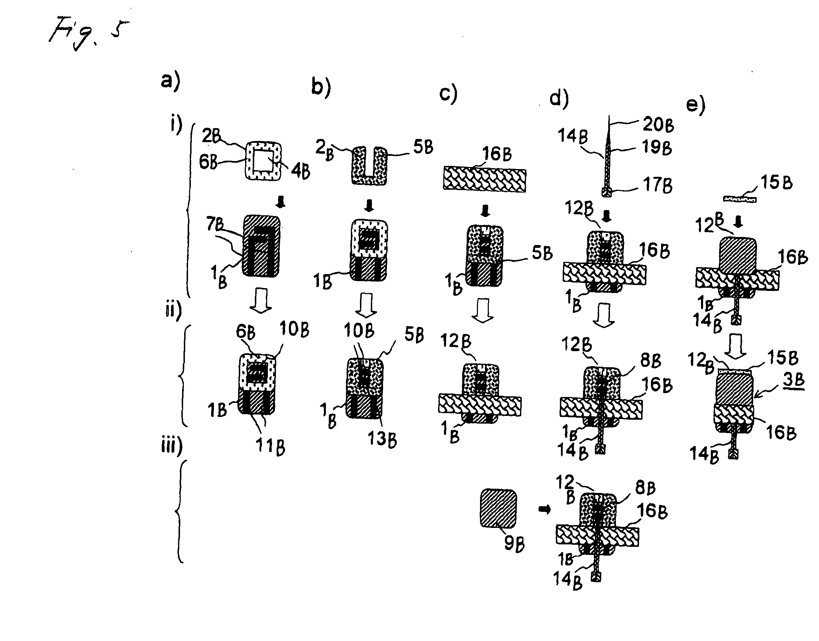

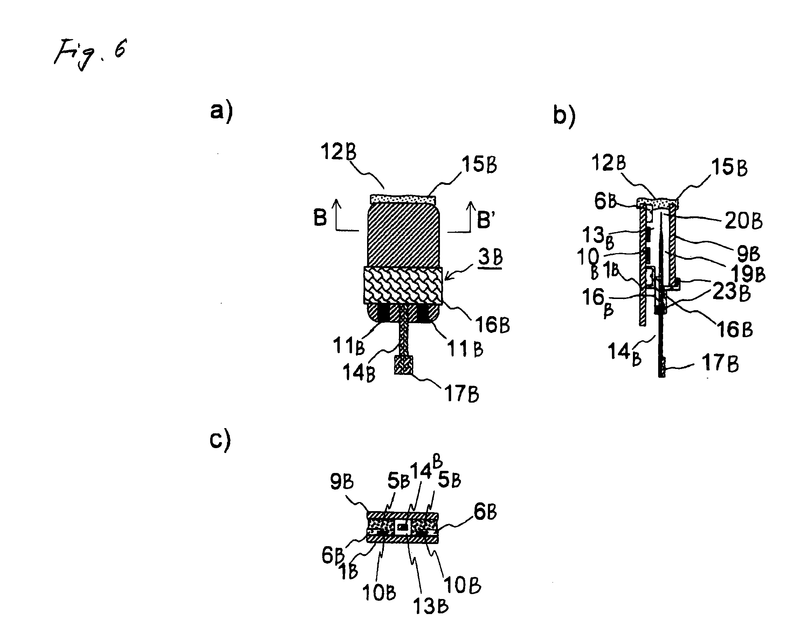

Fig. 5 shows an assembling example of the needle integrated biosensor according to the present invention. Figs. 5a) to e) are manufacturing examples of the needle integrated biosensor in which i) and iii) show constituting materials needles for the manufacture of the needle integrated biosensor while ii) shows its molding. Fig. 5a) shows a resist layer 6B and a plate material where an electrically conductive material 7B is formed on the surface of the lower substrate 1B of the biosensor. The resist layer 6B plays a role of a spacer 2B and, further, it is also installed for regulating the electrode area and to prevent the contact of the electrode surface to the puncture needle. Therefore, a penetration hole 4B is formed on the resist layer 6B. Here, the lower substrate 1B and the upper substrate 9B are safely used by rounding the corners. Fig. 5b) shows the state where an adhesive layer 5B is formed on the resist layer 6B. Here, the adhesive layer 5B is also formed between the plate materials of the lower substrate 1B and the upper substrate 9B and, therefore, it plays a role of a spacer 2B as same as the resist layer 6B. Fig. 5b)ii) shows the electrode 10B where its area is regulated by the resist layer 6B and the adhesive layer 5B as well as the electrode reaction part 13B thereof. Fig. 5c) shows a state where a shrinkable material 16B is formed so as to cover the lower part of the adhesive layer 5B. As shown in Fig. 5d), the shrinkable material 16B adheres to each of the puncture needle part 14B and the lower substrate 1B and the upper substrate 9B is covered by such a state whereby, as shown in Fig. 5e), each of the puncture needle part 14B and the upper substrate 9B is covered thereby and, finally, the inner area of the puncture needle path 8B forming a sample-conveying path is shut out from outer air. As shown in Fig. 5d), the puncture needle part 14B is constituted from a puncture needle 20B, a support 19B which supports it and a connecting part 17B for an outer driving and there is an arrangement that, when the connecting part 17B to an outer drive is connected to a measuring apparatus equipped with a piercing drive, a piercing drive from the measuring apparatus is achieved. It is also noted from Fig. 6) that the puncture needle part 14B is aligned in parallel to the long-axis direction of the electrode 10B. As shown by the drawing, the puncture needle part 14B has such a structure that its contact to the electrode surface 10B is able to be avoided by the formation of the resist layer 6B. Further, in Fig. 5e), there is a structure where the air tightness in the inner area of a sensor is able to be retained by forming a layer of a soft material 15B near the opening 12B for piercing and collecting the blood by piercing of the needle integrated biosensor 3B.

Fig. 6 shows a constitution example, by way of a cross-sectional view, of the needle integrated biosensor 3B shown in Fig. 1. Fig. 6b) shows a longitudinal cross-sectional view along a central line shown in Fig. 2a). As shown in the drawing, a puncture needle part 14B is aligned on the surface of a pattern formed on the lower substrate 1B of the biosensor. The puncture needle part 14B is further fixed by a shrinkable material 16B to the lower substrate 1B and the upper substrate 9B via an adhesive part 23B. Fig 6c) shows a cross-sectional view along the line B-B' shown in Fig. 6a). A puncture needle part 14B is placed at the central part of the lower substrate 1B and the upper substrate 9B.

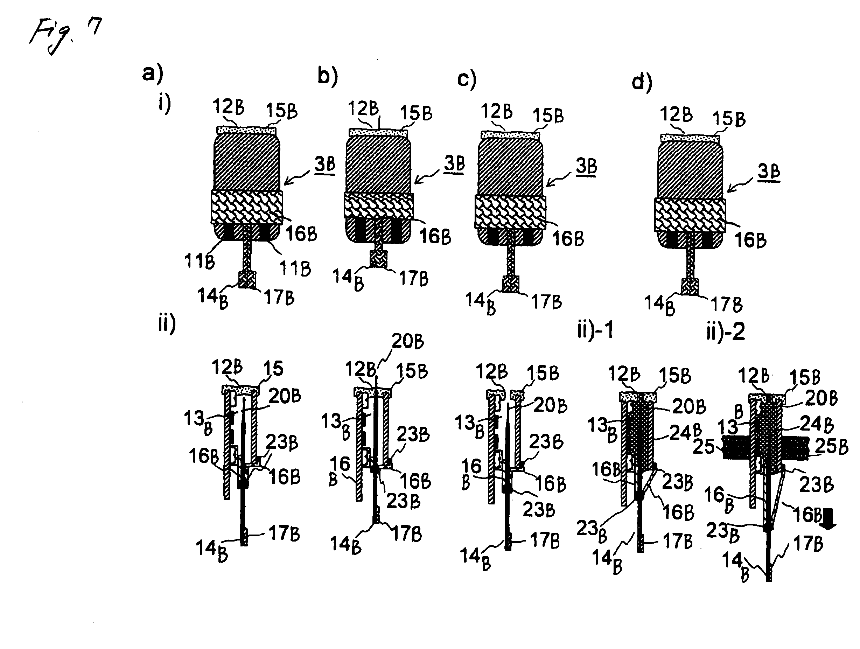

Fig. 7 shows a use example of the needle integrated biosensor 3B as shown in Figs. 5 and 6. In Fig. 7, each step is shown by a) to d) and, in i) and ii), state of the needle integrated biosensor 3B at that is shown where i) is a constitution drawing while ii) is a longitudinal cross-sectional view along the central line of the needle integrated biosensor shown in i). Fig. 7a) shows the state of the needle integrated biosensor 3B connected to the measuring apparatus with a piercing drive before actual use. At that time, the skin as a thing to be tested is closely attached to the soft material 15B installed in an opening 12B for piercing and collecting the blood of the needle integrated biosensor 3B. Fig. 7b) shows the state of piercing and shows the state when the puncture needle 20B penetrates the soft material 15B. Although not shown in the drawing, the puncture needle 20B also pierces the skin at that time. The state where the shrinkable material 16B shrinks is also noted. Fig. 7c) shows the state where the puncture needle part 14B returns to the original position after the piercing. Here, a soft material 15B penetrated by the puncture needle 20B is shown. In this state, negative pressure in the biosensor is applied to the pierced skin. Fig. 7d) shows the state where, after that, the collected blood 24B from the pierced skin is sucked by means of negative pressure in the inside (ii-1). Fig. 7d)ii)-2 shows the state where both sides of the needle integrated biosensor 2B are pushed and fixed by a pushing device 25B of the measuring apparatus whereby the puncture needle part 14B of the needle integrated biosensor 3B in a state of ii-1 is able to be pulled in the direction which is opposite to the piercing direction whereby the inner area of the sensor is made in more negative pressure. It is noted that, at that time, the shrinkable material 16B is extended.

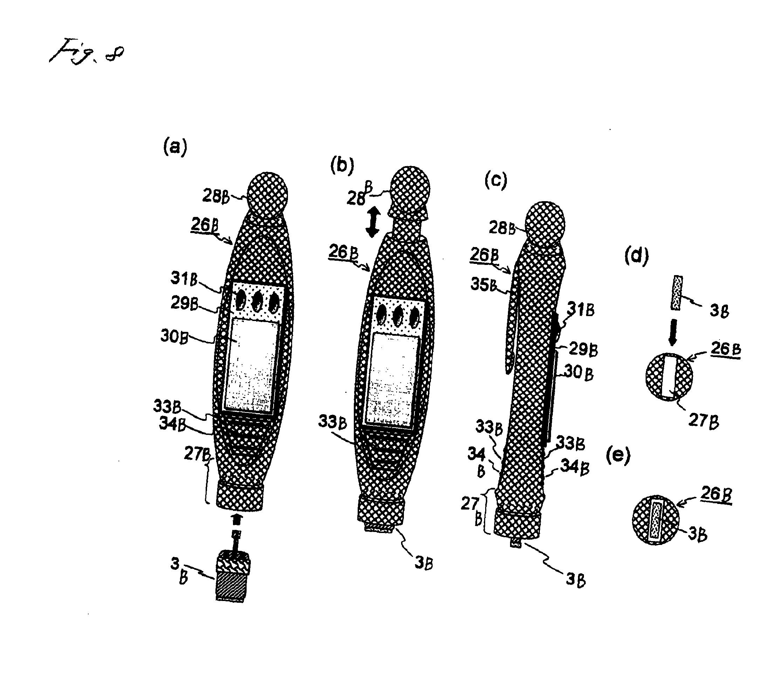

Fig. 8 shows an example of a measuring apparatus 26B equipped with a piercing drive which is able to be equipped with the needle integrated biosensor of the present invention. Fig. 8a) shows the state before the needle integrated biosensor 3B is introduced into a measuring apparatus 26B. The measuring apparatus will be illustrated using this drawing. The measuring apparatus 26B is constituted from an introducing part 27B for the needle integrated biosensor 3B, a trigger part 28B for a piercing drive, an operation panel 29B and a piercing initiation button 33B. A display part 30B and an operation button 31B are formed on the operation panel 29B while, on the piercing initiation button 33B, a sliding preventer 34B is formed. Fig. 8(b) shows the state where a sensor 3B is introduced into the measuring apparatus 26B and a trigger 28B on the upper area is pulled whereby the electric source of the measuring apparatus is made on so that a measuring mode is resulted. Fig. 8(c) shows the state after the sensor 3B is introduced into the measuring apparatus 26B from a lateral direction. As the drawing shows, a hook 35B is formed on the back of the display part whereby the measuring apparatus itself is easily able to be received in a breast pocket or an inside pocket. Fig. 8(d) shows the state where the sensor 3B and the measuring apparatus 26B are seen from the lower position while Fig. 8(e) shows the case where the sensor 3B is inserted into the measuring apparatus 26B from a lower side. The sensor 3B is that which is shown in Fig. 5. Example 3

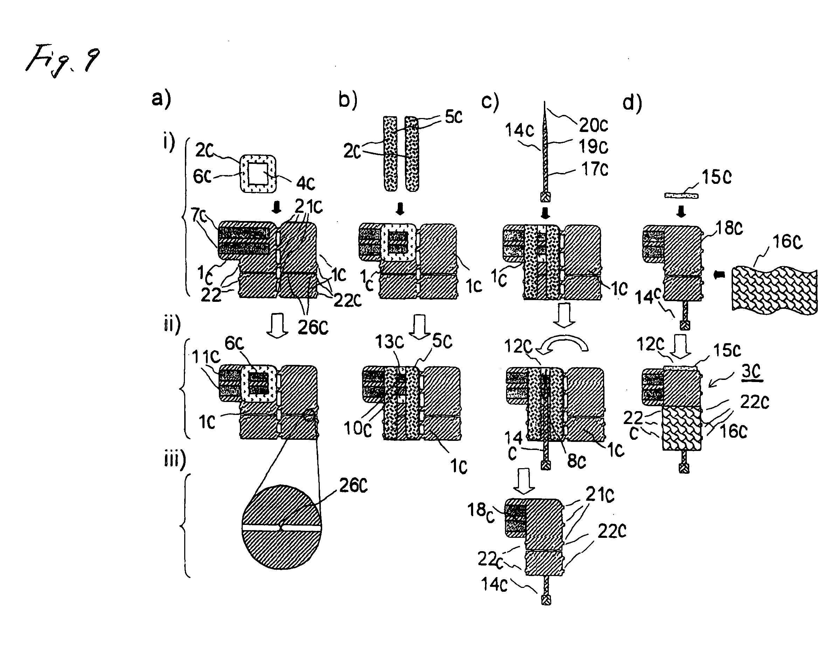

Fig. 9 shows an example of assembling of the needle integrated biosensor according to the present invention. Figs. 9a) to d) are examples for the manufacture of the needle integrated biosensor, i) shows constituting materials for the manufacture of the needle integrated biosensor and ii) and iii) show moldings thereof. Fig. 9a) shows a connecting part 21C by perforations between the substrates 1C and 1C of the biosensor where cutting part 26 is formed, a product where electrically conductive materials 7C and 7C are formed on the surface of one substrate and a resist layer 6C. With regard to the cutting part, that which is 1 x 1 mm and has a constriction in its center is formed on a substrate part containing no electrode in two places. The resist layer 6C plays a role of a spacer 2C and, moreover, it is also formed with the purpose of regulating the electrode area and of preventing the contact of the electrode surface to the puncture needle. Accordingly, a penetration hole 4C is formed in the resist layer 6. Here, the substrate 1 is able to be safely used by rounding its corners.

Fig. 9c) shows the state where an adhesive layer 5C is formed on a resist layer. Since an adhesive layer 5C is also formed between the substrates 1C and 1C, it plays a role of a spacer 2C the same as the resist layer 6C. Fig. 9b)ii) shows an electrode 10C where its area is regulated by a resist layer 6C and an adhesive layer 5C and also shows an electrode reaction part 13C thereof. Fig. 9c)i) shows the constitution of a puncture needle part 14C where the puncture needle part 14C is constituted from a puncture needle 20C, a support which supports the needle and a connecting part 17C to the outer drive and, when the connecting part 17C to the outer drive is connected to the measuring apparatus equipped with a piercing drive, a piercing drive from the measuring apparatus is available. In Fig. 9c), it is noted that the puncture needle part 14C is aligned at right angles to the electrode along the sample-conveying path 8C. As the drawing shows, the puncture needle part 14C has such a structure that the contact of it to the electrode surface 10C is prevented by the formation of the resist layer 6C. Therefore, even if a reagent layer 14C is formed on the electrode surface 10C, contact of the reagent layer 13C to the puncture needle part 14C is able to be avoided and, as a result, pollution of the puncture needle 20C with a reagent is able to be prevented. Fig. 9c)iii) shows the needle integrated biosensor 3C formed as such.

When a connecting part such as perforation is formed between the substrates 1C and 1C, a biosensor as a molding 18C is assembled. Firstly, unlike a manufacture method by layering, the biosensor assembled by such a folding system does not need layering of substrates whereby there is an advantage that the manufacturing steps can be simplified. Accordingly, it is a method which is suitable for the production of a sensor which is formed with high precision in a large scale in a high yield. The connecting part 21C for the substrates which is necessary for the folded structure also acts as a hook 22C for fixing the shrinkable material 16C to the substrate as shown in Fig. 9d)ii).

Fig. 9d) shows the state where a sample-conveying path and a piercing drive part 8 are shut out from the outer air by a shrinkable material 16 which is to tightly close the space among the puncture needle support 17C, the substrate 1C and the soft material 15C to the place near the opening 12C for piercing and collecting the blood. The shrinkable material 16C also covers the cutting part. The soft material is also helpful for a close adhesion of the skin of the person to be tested with the opening 12C for piercing and collecting the blood.

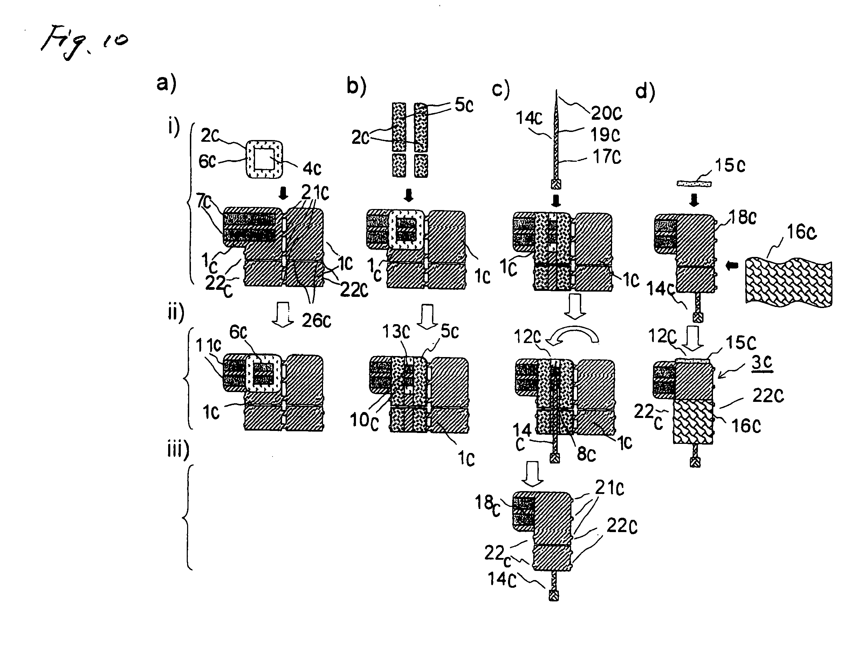

Fig. 10 shows another constitution example of the needle integrated biosensor according to the present invention. Figs. 10a) to d) are examples of manufacture of the needle integrated biosensor in which i) shows constituting materials needed for the manufacture of the needle integrated biosensor and ii) and iii) show the moldings thereof. Difference from the needle integrated biosensor shown in Fig. 9 is that the adhesive layer 5C is formed in a mode of avoiding the cutting part. As compared with the case of Fig. 9, the biosensor assembled by such a manner also has a characteristic that separation of the substrate by cutting is easy.

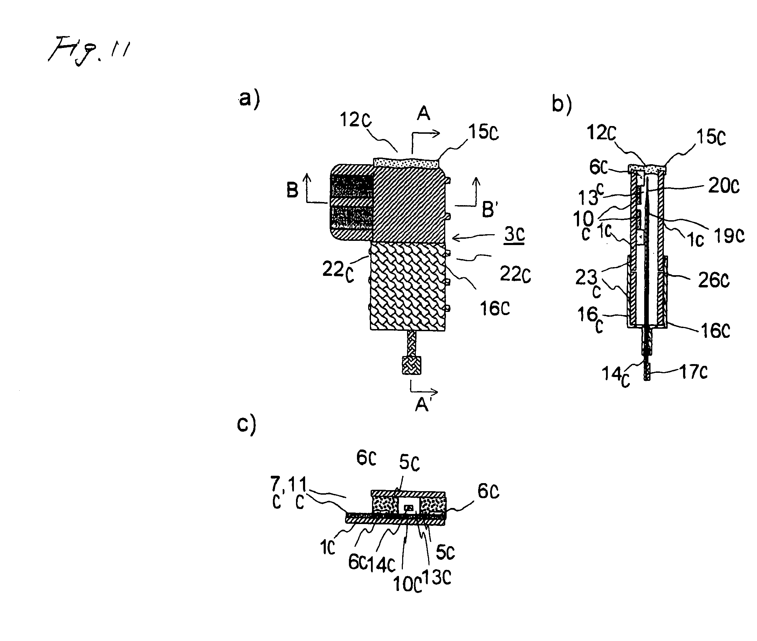

Fig. 11 shows the cross-sectional view of the needle integrated biosensor 3C shown in Fig. 9 or Fig. 10. Fig. 11b) shows a cross-sectional view thereof along the line A-A' of Fig. 3a). As the drawing shows, a puncture needle 14C is placed on the pattern surface formed on the substrate 1C of the biosensor. Fig. 11c) shows a cross-sectional view thereof along the line B-B' of Fig. 3a). As shown in Fig. 11b), the cutting part 26C is covered by a shrinkable material 16C. As those drawings show, in the needle integrated biosensor 3C according to the present invention, a puncture needle 14C is arranged at right angles to the long-axial direction of the electrodes 10C and 10C formed in the inner area of one substrate 1C whereby the terminal 11C is able to be parted from the orbit of the puncture needle 14C. Further, since the terminal 11C is aligned at the position apart from the orbit of the puncture needle 14C, the shape of the needle integrated biosensor 3C becomes bilaterally asymmetric where the puncture needle is a central line whereby, for a user, the above acts as a mark so that a wrong insertion into the measuring apparatus in right or left is able to be prevented while the measuring apparatus itself is also able to be equipped with a mechanism for specifying the position of the terminal 11C of the needle integrated biosensor 3C according to the present invention. Moreover, when the width of the electrode and the distance between electrodes are made small, the width of the substrate there is also able to be made small whereby the amount of the liquid sample is made small.

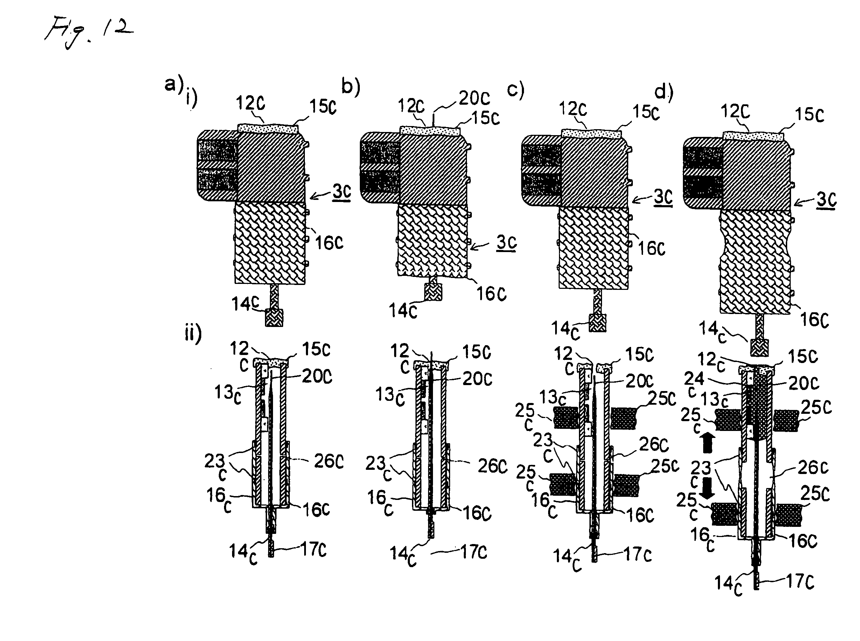

Fig. 12 shows a use example of the needle integrated biosensor 3C shown in Figs. 9 and 10. In Figs. 12, a) to d) show each of the steps where i) and ii) show the state of the needle integrated biosensor 3C in which i) is a constitution drawing while ii) is a cross-sectional view along the line A-A' shown in Fig. 11a). Fig. 12a) shows the state before actual use of the needle integrated biosensor 3C connected to the measuring apparatus equipped with a piercing drive. At that time, the skin as a thing to be tested is closely adhered to the opening 12C for piercing and collecting the blood of the needle integrated biosensor 3C. Fig. 12c) shows the state of piercing and, although not shown in the drawing, the puncture needle 20C is projected from the sensor and pierces the skin. Fig. 12c) shows the state where the puncture needle part 14 after the piercing returns to the original position. Fig. 12d) shows the state that, after the above, the cutting part 25C is separated and the inner volume of the sensor is increased whereby the inner part of the sensor is made in negative pressure. It is noted that, at that time, the shrinkable material 16C is stretched. As a result, the blood 24C collected from the pierced skin is sucked and collected into the inner area of the sensor. At that time, when both sides of the needle integrated biosensor 3C are pushed by a pushing device 25C of the measuring apparatus and fixed, the puncture needle part 14C is able to be pulled in the direction which is opposite to the piercing direction and, as a result, the cutting part is separated and the shrinkable material 16C covering the cutting part is extended whereupon the inner part of the biosensor becomes a negative pressure.

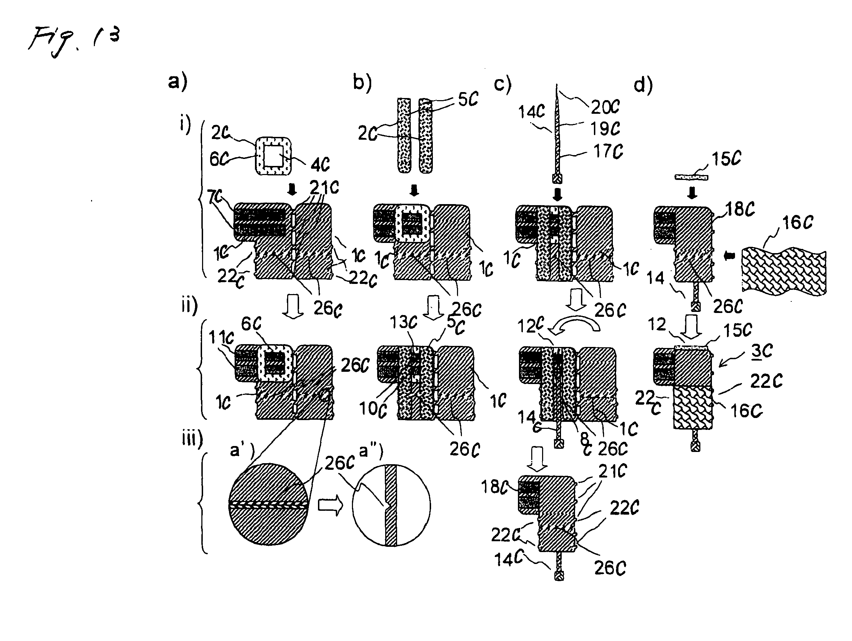

Fig. 13 shows still another constituting example of the needle integrated biosensor of the present invention. Fig. 13a) to d) are examples of manufacture of the needle integrated biosensor in which i) shows constituting materials needed fro the manufacture of the needle integrated biosensor while ii) and iii) are the moldings thereof. Difference from the needle integrated biosensor shown in Fig. 9 is that, as shown in the enlarged drawing a') and the cross sectional view thereof a') of Fig. 13a)ii), the cutting part 26C is formed as a groove (in a V-shape) formed in the depth which does not penetrate the substrate. As compared with the case of Fig. 9, in the biosensor assembled by such a manner, the cutting part 26C is not in such a manner that the bond between the substrates is not intermittently formed but is formed as a groove or, in other words, in a non-intermittent manner whereby it is hardly affected by, for example, deformation in the case of non-use. In addition, in the case of cutting of the cutting part 26 in the measuring apparatus shown in Fig. 12c)ii) and Fig 12d)ii), the cut-and-separating operation of the substrates is conducted from the direction at completely right angles to the direction of the groove of the cutting part whereby there is a characteristic that the force is applied uniformly to the whole groove and the substrates are cut.

Although the present invention is illustrated in detail by referring to specific embodiments hereinabove, it is apparent for persons skilled in the art that various changes and modifications thereof are able to be done without departing the spirit and the scope of the present invention.
The present application is on the basis of a Japanese patent application (Application No. 2005-185988) filed on June 27, 2005, a Japanese patent application (Application No. 2005-201072) filed on July 11, 2005 and a Japanese patent application (Application No. 2005-218685) filed on July 28, 2005 and their contents are incorporated herein as references.

## Claims

1. A needle integrated biosensor, comprising:
a biosensor including at least two electrodes, and
a puncture needle to collect a body fluid by piercing a skin of a person to be tested,
the biosensor and the puncture needle being integrated, wherein
a soft material is attached to a front end of the puncture needle.

2. The needle integrated biosensor according to claim 1, wherein
the soft material is sterilized.

3. The needle integrated biosensor according to claim 1, wherein
the soft material to which an antibiotic action is endowed is used.

4. The needle integrated biosensor according to claim 1, wherein
a space between two substrates and a puncture needle support for fixing the puncture needle to a substrate is tightly closed by a shrinkable material at a rear part of the puncture needle,
an inner area of the biosensor is kept in negative pressure, and
the puncture needle pierces both of the soft material and the skin to collect blood by means of suction and measure the detected component.

5. A needle integrated biosensor, comprising:
a biosensor including electrode and spacer and a puncture needle to collect a body fluid by piercing a skin of a person to be tested aligned in the biosensor are integrated via a puncture needle support in a space between two electrically insulating substrates, wherein
a piercing and blood-collecting opening at the front end of the puncture needle is tightly closed by a puncturable soft material while, in the rear end of the puncture needle, space between two substrates and the puncture needle support is tightly closed by a shrinkable material and the inner area of the biosensor is kept in a negative pressure, and
the blood is collected by means of suction and the components to be detected are measured when the puncture needle breaks through both of the soft material and the skin by means of a drive from outside.

6. The needle integrated biosensor according to claim 5, wherein
the puncture needle is placed in parallel to a long-axial direction of the electrode in the biosensor.

7. The needle integrated biosensor according to claim 5, wherein
if the puncture needle support is pulled in the direction opposite to the piercing direction immediately after the piercing,
the shrinkable material placed among the rear end of the puncture needle, the biosensor substrate and the cover is extended so that the inner part of the biosensor is made in more negative pressure.

8. A needle integrated biosensor comprising:
a biosensor including an electrode and a spacer in a space sandwiched between two sheets of electrically insulating substrate, and
a puncture needle which is provided in the biosensor to collect a body fluid by piercing a skin of a person to be tested,
the biosensor and the puncture needle being constituted in a integrated manner via a support for the puncture needle, wherein
each of the substrates includes a cutting part by which separation of substrate part where no electrode is formed,
a piercing and blood-collecting opening at the front end of the puncture needle is tightly closed by a puncturable soft material, cutting part of the two substrates are covered by a shrinkable material provided at the rear end of the puncture needle, and the space between the two substrates and the puncture needle is tightly closed so that the inner area of the biosensor is tightly closed, and
after the puncture needle simultaneously breaks through both of the soft material and the skin by being driven from outside, a part of the substrates is separated at the cutting part and, the volume of the inner area of the biosensor is increased to make the inner area in negative pressure to collect the blood by means of suction and measure the components to be detected.

9. The needle integrated biosensor according to claim 8, wherein
the cutting part is constituted from an easily cuttable bonding part.

10. The needle integrated biosensor according to claim 9, wherein
the easily cuttable binding part is formed as at least one bonding part on a line between the substrates, or is constituted by a groove formed in a depth which does not penetrate the substrate at least on one side of the substrate.

11. The needle integrated biosensor according to claim 10, wherein
a linear bonding part where the central area is constricted is used.

12. The needle integrated biosensor according to claim 10, wherein
a V-shaped groove is used.

13. The needle integrated biosensor according to claim 8, wherein
the puncture needle is provided in a crossing manner in a long-axial direction of the electrode formed on one of the substrates.

14. The needle integrated biosensor according to claim 8, wherein
the puncture needle is provided in a crossing manner at a right angle to the long-axial direction of the electrode formed on one of the substrates.

15. The needle integrated biosensor according to claim 8, wherein
the biosensor has a bilaterally asymmetric shape where the puncture needle is a central line.

16. The needle integrated biosensor according to any one of claims 1, 5 and 8, wherein
the soft material is a gel-like material, an elastic material or a foamed material.

17. The needle integrated biosensor according to claim 16, wherein
the elastic material is silicone rubber.

18. The needle integrated biosensor according to any of claims 4, 5 and 8, wherein
the shrinkable material is natural rubber.

19. The needle integrated biosensor according to claim 8, wherein
if a puncture needle support is pulled immediately after piercing in a direction opposite to the piercing direction,
the cutting part provided in the biosensor substrate is cut off, and volume of the inner part of the biosensor is increased so that the inner part is made in a negative pressure.

20. A measuring apparatus for a needle integrated biosensor, comprising:
the needle integrated biosensor according to any one of claims 1 to 19,
an introducing part for the needle integrated biosensor where the needle integrated biosensor is inserted and set,
a connector part for catching electric signal at an electrode of the needle integrated biosensor,
a measuring part for measuring an electric value via the connector part,
an operation panel part for the measurement,
a display part for displaying a measured value at the measuring part,
a memory part for storing the measured value,
a driving part for driving a puncture needle, and
a pierce starting button for starting the piercing by a drive trigger part and the puncture needle.

21. A measuring apparatus for the needle integrated biosensor according to any one of claims 5 to 7, comprising:
a piercing drive, and
an extension mechanism of a shrinkable material connecting the puncture needle and the biosensor.

22. A measuring apparatus for the needle integrated biosensor according to any one of claims 8 to 19, comprising:
a piercing drive, and
a cutting part for a biosensor substrate and a cutting off mechanism.

23. A measuring apparatus for the needle integrated biosensor according to any one of claims 8 to 19, comprising:
a mechanism to recognize the bilaterally asymmetric structure where the puncture needle for the needle integrated biosensor is a central line at a projected part of a terminal for the measurement.

24. The measuring apparatus for a needle integrated biosensor according to claim 20, comprising:
at least one of voice guidance mechanism and a voice recognition mechanism, a control mechanism for measured data by incorporation of radio clock, a communication mechanism of the measured data to medical organizations and a charging mechanism.
